(19) Europäisches Patentamt
European Patent Office
Office européen des brevets

(11) Publication number: **0 030 393**
**B1**

(12) # EUROPEAN PATENT SPECIFICATION

(45) Date of publication of patent specification: **15.06.83**

(21) Application number: **80200884.7**

(22) Date of filing: **19.09.80**

(51) Int. Cl.³: **C 12 N 9/24, C 12 P 19/14 //C12R1/15**

(54) **Xanthanase enzyme and its production.**

(30) Priority: **26.10.79 GB 7937169**

(43) Date of publication of application:
**17.06.81 Bulletin 81/24**

(45) Publication of the grant of the patent:
**15.06.83 Bulletin 83/24**

(84) Designated Contracting States:
**BE CH DE FR GB IT LI NL**

(56) References cited:
**LU - A - 65 867**

**CHEMICAL ABSTRACTS, vol. 88, no. 15, 10th April 1978, page 233 abstract 101289y Columbus, Ohio, US A.C. HAYWARD: "Occurrence of glycoside hydroglases in plant pathogenic and related bacteria"**

**CHEMICAL ABSTRACTS, vol. 85, no. 3, 19th July 1976, page 509, abstract 19078h Columbus, Ohio, US**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Cripps, Roger Edward**
**Carel van Bylandtlaan 30**
**NL-2596 HR The Hague (NL)**
Inventor: **Sommerville, Hugh John**
**Carel van Bylandtlaan 30**
**NL-2596 HR The Hague (NL)**
Inventor: **Holt, Martin Stafford**
**69 Sterling Road**
**Sittingbourne Kent (GB)**

(74) Representative: **Rogers, Roy Charles et al,**
**4 York Road**
**London SE1 7NA (GB)**

**CHEMICAL ABSTRACTS, vol. 61, no. 12 7th December 1964, column 15056f Columbus, Ohio, US D.C. HILDEBRAND et al.: "Beta-Glucosidase activity in phytopathogenic bacteria"**

**EP 0 030 393 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

# Xanthanase enzyme and its production

This invention relates to a process for the microbial production of an enzyme which depolymerises xanthan gum, the enzyme thereby produced and the application of that enzyme in the depolymerisation of xanthan gum.

Xanthan gum, and other polysaccharides such as carboxymethyl cellulose and tragacanth are used in a variety of commercial applications, for example xanthan gum is used in the commercial extraction of oil where they may be used to increase the viscosity of drilling muds and secondary extraction fluids. However, the increased viscosity can often make subsequent processing more difficult, and it would therefore be advantageous if, after use, the xanthan (or other polysaccharide) could be depolymerised, thereby reducing its viscosity. The Applicants have now discovered that such a depolymerisation can be achieved by a new enzyme produced by a novel strain of *Corynebacterium.*

Accordingly, the present invention provides a process for the production of a xanthan gum depolymerising enzyme (hereinafter termed "xanthanase") which comprises culturing *Corynebacterium* strain 20/122 (deposited with NCIB) under accession number 11535) under aerobic conditions in a liquid growth medium comprising assimilable sources of nitrogen and essential mineral salts and, as the sole carbon source, xanthan gum, separating the bacterial cells from the reaction mass and recovering the xanthanase from the resultant spent growth medium.

The *Corynebacterium* 20/122 was isolated by inoculating with soil samples a series of sterile liquid enrichment culture media having the composition shown in Table I below:—

TABLE I

| *Component* | *Concentration ($1^{-1}$)* |
|---|---|
| Xanthan | 1.0 g |
| $Na_2HPO_4$ | 3.0 g |
| $KH_2PO_4$ | 3.0 g |
| $(NH_4)_2SO_4$ | 0.3 g |
| $MgSO_4.7H_2O$ | 0.2 g |
| $FeCl_3.6H_2O$ | 16.7 g |
| $CaCl_2.2H_2O$ | 0.66 g |
| $ZnSO_4.7H_2O$ | 0.18 mg |
| $CuSO_4.5H_2O$ | 0.16 mg |
| $MnSO_4.4H_2O$ | 0.15 mg |
| $CoCl_2.6H_2O$ | 0.18 mg |
| $H_3BO_3$ | 0.10 mg |
| $Na_2MoO_4.2H_2O$ | 0.30 mg |

If the complete medium was sterilised by autoclaving, degradation of the xanthan gum occurred, as judged by loss of viscosity. Thus, in practice separate solutions of the xanthan and of the salts, made up at double the final strength, were autoclaved separately and equal volumes of the sterile solutions mixed to give the complete medium. The xanthan solution was autoclaved at 100°C for 25 minutes and the salts solution at 110°C for 25 minutes. Inoculated growth medium was incubated at 30°C on a rotary shaker until the medium had lost its viscosity and microbial growth was apparent. The bacterium was isolated and purified from this culture using conventional microbiological techniques by streaking onto agar plates containing the same medium solidified with 1.2% agar.

In practice, it was found that even the modified procedure adopted to autoclave the enrichment medium for the inoculum was not sufficient to guarantee sterility. Consequently, in order to prevent contaminated cultures, a streptomycin resistant mutant of the initially isolated organism was obtained by streaking that organism onto agar plates of the above medium containing 100 $\mu$g/ml of streptomycin sulphate. One of the resulting organisms that grew on these plates was designated

*Corynebacterium 20/122*, and has been deposited with NCIB under accession number 11535. All further work was carried out with this mutant strain and streptomycin sulphate at 100 $\mu$g/ml was always included in the growth medium. The antibiotic was included with the xanthan solution during the autoclaving procedure.

*Corynebacterium* 20/122 has the characteristics in Table II below. These were determined by standard test methods described in 'Cowan and Steel's Manual for the Identification of Medical Bacteria' second edition (1974). Comparison of these characteristics with those listed in 'Bergy's Manual of Determinative Bacteriology, Eighth Edition' reveals that the organism belongs to the genus *Corynebacterium* but insufficient data are available to ascribe it to a particular species; hence the designation, *Corynebacterium* 20/122.

TABLE II

*Characteristics of Corynebacterium 20/122*

1. *Physical characteristics*
   - (a) Shape: Pleomorphic, elongated rods to club shapes and coccal forms. Cells often arranged in a 'pallisade' in cultures. Granules evident when stained with Loeffler's Methylene Blue.
   - (b) Motility: Non-motile.
   - (c) Size: 0.5—2.5 $\mu$
   - (d) Sporulation: Non-sporulating.
   - (e) Gram's stain: Positive.

2. *Cultural characteristics*

   Nutrient Agar plate: Colonies off-white, circular, entire, low convex, smooth and soft. Diameter 0.5 mm after 24 hours at 30°C.

3. *Physiological characteristics*
   - (a) Catalase: Positive.
   - (b) Oxidase: Negative.
   - (c) Urease production: Positive.
   - (d) Oxygen relations: Aerobic.
   - (e) Temperature relations: Growth between 4°C and 42°C.
     Optimum at 30°C.
   - (f) pH relations: Growth between pH5 and 8.
     Optimum at pH6—7.
   - (g) Methyl Red test: Negative.
   - (h) Carbohydrate breakdown: Fermentative.
   - (i) Voges-Proskauer reaction: Negative.
   - (j) Nitrate reduction: Negative.
   - (k) Tween hydrolysis: Negative.
   - (l) Gelatin hydrolysis: Positive.
   - (m) Casein hydrolysis: Negative.
   - (n) Acid fastness: Negative.
   - (o) Pigment production: Kings A medium — pale yellow.
     Kings B medium — pale pink-orange.
   - (p) Indole production: Negative.
   - (q) $H_2S$ production: Negative.
   - (r) Utilisation of carbon sources: Growth on glucose, lactose, sucrose, xylose, maltose, arabinose and gluconate (poorly). Citric acid not utilised for growth. Acid produced from glucose, sucrose, xylose, maltose and arabinose.
   - (s) Antibiotic sensitivity: Tetracyclin, Erythromycin, Lincomycin.
   - (t) Antibiotic resistance: Penicillin G, Streptomycin.

The liquid growth medium used in the process of the invention comprises a nitrogen-containing compound which may, for example, be ammonia, urea, an ammonium salt such as a sulphate or chloride, or a nitrate, for example an alkali metal nitrate. The compound is suitably present in a concentration from 1—50 g/l.

Other elements which should desirably be present in the medium are phosphorus, sulphur, magnesium and iron. The phosphorus source is preferably one or more phosphates, for example $K_2HPO_4$, $KH_2PO_4$, $Na_2HPO_4$ or $(NH_4)_2HPO_4$, or phosphoric acid, preferably present in a concentration from 1—20 g/l. The sulphur source may be sulphuric acid or a sulphate such as $(NH_4)_2SO_4$ suitably in a concentration from 0.1—5.0 g/l. The two metals are provided as one of their salts, for example $MgSO_4.7H_2O$ in a concentration from 0.1—2.0 g/l and $FeCl_3.6H_2O$ in a concentration from 5—50 mg/l. The medium may also contain trace amounts of other elements in the form of suitable salts, for example calcium, manganese, zinc, cobalt, molybdenum and boron.

The incubation of the inoculated culture is conveniently carried out by shaking under temperature and pH conditions controlled to produce the desired xanthanase with maximum yield, namely at a temperature between 25°—40°C, preferably 30°C, and a pH between 5 and 8, preferably between 6.0 and 7.0. The incubation was continued until the protein concentration in the growth medium had reached a maximum, which normally was achieved after 40—48 hours. The bacterial cells were then removed, suitably by centrifugation, and the xanthanase recovered from the spent growth medium, suitably by precipitation with, for example, ammonium sulphate or ethanol. The precipitate could then be redissolved in a small volume of water (suitably 100 ml of water per litre of original culture) and residual precipitating agent removed by dialysis against distilled water, followed by final concentration by freeze-drying. Alternatively, the xanthanase could be recovered by freeze-drying the spent growth medium from which the cells had been removed.

The invention includes also the xanthanase enzyme produced by the foregoing process, which enzyme has been characterised by the reaction products derived from the enzymic depolymerisation of deacetylated xanthan gum. There are 4 major products, which have Rf values of 0.55, 0.50, 0.14 and 0.05 when resolved by chromatography on cellulose thin layer plates using, as solvent, ethyl acetate-pyridine-glacial acetic acid — water (5:5:1:3 by volume), and detected with standard silver nitrate-sodium hydroxide spray reagent. The four products were further identified by sugar analysis as being, in order of decreasing Rf value, mannose, mannose ketal-linked to pyruvate, an oligosaccharide containing glucose, mannose and uronic acid in the proportions 1.0:0.5:0.34 respectively, and an oligosaccharide containing glucose, mannose and uronic acid in the proportions 1.0:0.84:0.55.

The invention further includes a process for the depolymerisation of xanthan gum or carboxymethyl cellulose (CMC), in which an aqueous solution of the gum or CMC is reacted with the xanthanase produced as defined above at a temperature between 25°C and 70°C, preferably 30°C, and a pH between 5 and 7, preferably pH 5.6.

The invention is further illustrated in the following Examples.

Example 1

*Corynebacterium* 20/122 was inoculated into 200 ml of medium contained in a 1 l flask. The medium was as defined in Table I above, with the addition of 100 μg/ml streptomycin sulphate, and had a pH of 6.8. The inoculated medium was incubated at 30°C on a reciprocating shaker, and growth of the bacteria was monitored by optical density measurements at 625 nm and by following the decrease in viscosity of 1 ml samples of the broth in a Wells-Brookfield microviscometer. After 48 hours the concentration of xanthanase enzyme protein in the growth medium had reached its maximum as determined by enzyme activity (see below), and the cells then removed by centrifugation and filtration of the clear supernatant through 0.22 μm Millipore filters.

The xanthanase enzyme was then recovered from the resulting cell-free spent growth medium by the addition, with stirring, of solid, recrystallised ammonium sulphate in an amount sufficient to give a final ammonium sulphate concentration of 70% complete saturation. Alternatively, ethanol can be added to give a final concentration of 60%. The precipitate was collected by centrifugation (filtration being an alternative procedure), redissolved in a small volume of water (approx. 100 ml per litre of original culture), and residual ammonium sulphate or ethanol removed by dialysis against three changes of distilled water at 4°C. Final concentration of the xanthanase enzyme was then achieved by freeze-drying, to yield a product whose activity could be demonstrated either by following the loss of viscosity over a 10 minute period at 40°C of 1 ml samples of a 1 g/l solution of xanthan gum in water following addition of 0.1 ml enzyme solution, using a Wells-Brookfield LVT microviscometer, or by the release of reducing sugars over a 10 minute interval at 30°C from a mixture containing, enzyme and 1 mg of xanthan gum, in 2 ml of 25 millimolar acetate buffer pH5.6. (Reducing sugar was measured by the method of Nelson (Journal of Biological Chemistry *153*, 375 (1944))).

The enzyme has been established as being stable for at least 3 months in distilled water at 0°C. Its activity is optimal at pH5.6, and a temperature of 70°C. However, the activity diminishes at elevated temperature; over a one hour period, 30% of activity is lost at 30°C, 50% lost at 45°C, and 100% (i.e. total inactivation) at 50°C.

Example 2

200 mg of xanthan gum in 200 ml of distilled water were incubated at 30°C with 0.5 mg of the xanthanase enzyme produced according to Example 1 above for a period of 15 hours with shaking. A few drops of chloroform were added to the mixture to prevent microbial growth. Under these conditions the viscosity of the solution (as measured with 1 ml samples in a Wells-Brookfield microviscometer) rapidly decreased, thereby establishing the degradation (or depolymerisation) of the xanthan gum.

Similar experiments carried out with carboxymethyl cellulose established that the xanthanase enzyme was also effective in depolymerising that material, its activity being 2½ times greater than its activity with xanthan gum. In the case of other commercial polysaccharide gums such as tragancanth and carrageenan, the enzyme showed a rapid initial activity, but this soon diminished before a significant extent of depolymerisation had been achieved.

Example 3

The end products from the xanthan gum depolymerisation described in Example 2 above, and of similar treatment of a deacetylated xanthan gum, were subjected to thin layer chromatography and sugar analysis in order to identify their structure and thence the mode of action of the xanthanase enzyme.

The depolymerised end product of Example 2 was evaporated to dryness under reduced pressure at 40°C and the residue dissolved in water. The products of the reaction in this solution were resolved by chromatography on cellulose thin layer plates using, as solvent, ethyl acetate — pyridine — glacial acetic acid — water (5:5:1:3 by volume), being detected on the chromatograms with a silver nitrate-sodium hydroxide spray reagent (Data for Biochemical Research, Editors R. Dawson, D. Elliott, W. Elliott, K. Jones; second edition, Oxford at the Clarendon Press, 1969. p.541).

At least nine reaction products were obtained from xanthan gum using this procedure. However, if the xanthan was first deacetylated by treatment under a nitrogen atmosphere for 2 hours at room temperature with 25 millimolar potassium hydroxide in the presence of 1% potassium chloride, and then depolymerised by the procedure of Example 2, only 4 major sugar containing reaction products were obtained. Several other materials that react with the silver nitrate spray are also seen on the chromatograms but only occurred in small quantities and were not further characterised. The Rf values of the major products in the above thin-layer chromatography system were 0.05, 0.14, 0.50 and 0.55.

The four products from deacetylated xanthan gum were separated, purified and characterised. Separation was obtained by chromatography on paper as described above and the 4 components were purified by gel permeation and ion-exchange chromatography using established procedures. They have been designated P1, P2, P3 and P4 on the basis of increasing Rf values in the above thin layer chromatography system.

These four products were then characterised with respect to their constituent sugars. Neutral sugars were determined after hydrolysis of the materials by gas chromatography after conversion to their aldononitrile acetate derivatives. The products (0.5 mg) were hydrolysed with 0.25 molar sulphuric acid (0.2 ml) at 95°C for 20 hours. The mixtures of sugars produced was neutralised with an ion-exchange resin (Bio-Rad Agl — X2, bicarbonate form) and evaporated to dryness with a stream of nitrogen. The sugars were converted to the aldononitrile acetates and analysed by gas chromatography as described by R. Varma and R.S. Varma (Journal of Chromatography *128*, 45—52 (1976)).

Uronic acids in the purified enzyme reaction products were determined by the carbazole reaction (T. Bitter and H.M. Muir (1962) Analytical Biochemistry *4*, 330—334) using glucuronic acid as a reference standard.

The enzyme product P4 was shown to be mannose by comparison with the authentic sugar by gas chromatography as the aldononitrile acetate in the system mentioned above and by thin layer chromatography on cellulose layers in the following solvents:

1. ethyl acetate-pyridine-glacial acetic acid-water (5:5:1:3 by volume).
2. propan-2-ol-ethyl acetate-water (6:1:3 by volume). Detection on the thin layer plates was by silver nitrate-sodium hydroxide and gave Rf values of 0.51 and 0.62 respectively. Authentic mannose behaved identically in both t.l.c. systems and by g.l.c.

The enzyme product P3 was hydrolysed and the only sugar released was shown to be mannose. When P3 was hydrolysed for 40 minutes at 100°C with 2 molar HCl and the hydrolysate neutralised to pH7.0 with sodium hydroxide, pyruvic acid was shown to be present. The acid was identified and determined by the use of lactate dehydrogenase as described by Bergmeyer (Methods of Enzymatic Analysis (1965) Academic Press, New York & London pp. 253—259). Approximately equimolar amounts of pyruvate and mannose were found in hydrolysates of P3. In view of the determined structure of xanthan gum (Jannson, Kenne and Lindberg (1975) Carbohydrate Research 45, 272—282; Melton, Mindt, Rees and Sanderson (1976) Carbohydrate Research *46*, 245—257), the analysis of P3 indicates that it is derived from the terminal residue of the trisaccharide side chain in which pyruvate is linked as a ketal to mannose. P3 may therefore be 4.6—0- (1-carboxyethylidene) mannose.

The enzyme product P2 was shown, after hydrolysis, to contain glucose and mannose in the molar ratio 1:0.50. Uronic acid was also detected by the carbazole test, the molar ratio of glucose to uronic acid (as glucoronic acid) being 1:0.34. However, the absorption spectrum of P2 showed a strong band at 232 nm and P2 also reacted strongly in the thiobarbituric acid test of Weissbach and Hurwitz (Journal of Biological Chemistry, *234*, 705—709 (1959)) indicating that the uronic acid was present as the unsaturated Δ 4,5 derivative, presumably as unsaturated Δ 4,5 glucuronic acid. The previous report that glucuronic acid and its Δ 4,5 unsaturated derivative give the same colour yield in the carbazole test has not been confirmed as no authentic sample of the unsaturated material was available. Hence it is not clear if the molar ratio of glucose to uronic acid in P2 of 1.0:0.34, as determined by the carbazole reaction using glucuronic acid as a standard, is precisely correct.

Further information on the structure of P2 was obtained by methylation analysis using the method of Hakomori as described by Lindberg (Methods in Enzymology, Vol. 28B, pp. 178—195 (1972)). Methylation analysis involves the replacement of all exposed hydroxyl groups in the oligosaccharide by methoxyl groups. When the modified oligosaccharide is subsequently hydrolysed to its constituent

(methylated) sugars, the newly exposed hydroxyl groups are derived from the breakage of the linkages between the sugar residues. Hence, analysis of the partially methylated sugars to determine the positions of the hydroxyl groups, indicates the positions in the sugar molecules involved in the linkages between sugars in the oligosaccharide. Analysis is carried out by combined gas-chromatography and mass spectroscopy.

The results with P2 showed that the methylation procedure resulted in only trimethyl derivatives of sugars, namely 3,4,6- 2,4,6- and 2,3,6- trimethyl hexoses. This indicates that P2 is an unbranched oligosaccharide (as no dimethyl sugars are formed) and that, as expected, the non-reducing terminal of the molecule is occupied by the unsaturated uronic acid residue (as no tetramethyl derivatives are formed and uronic acid derivatives are not recovered in this procedure). Hence, considering the published structure of xanthan gum, it appears that P2 is a tetrasaccharide having the molecular structure shown in formula I below:—

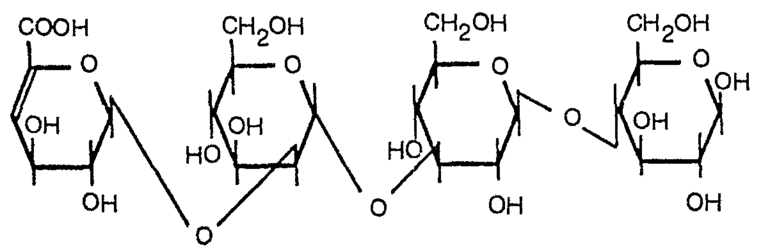

(I)

This structure would require a glucose:mannose:uronic acid molar ratio of 1.0:0.5:0.5 as opposed to the 1.0:0.5:0.34 determined.

P2 contains no pyruvic acid.

The enzyme product P1 was shown after hydrolysis to contain glucose, mannose and uronic acid in the molar ratio 1.00:0.84:0.55 P1 did not react in the thiobarbituric acid test nor did it absorb at 232 nm and therefore does not contain an unsaturated uronic acid. Methylation analysis indicated the formation of the same three trimethyl hexoses as in P2 and, in addition, 2,3,4,6-tetramethyl hexose was obtained. The tetramethyl derivative is derived from the non-reducing terminal of the oligosaccharide. Dimethyl sugars were again absent. Hence, P1 is believed to be an unbranched oligosaccharide having the molecular structure shown in formula II below:—

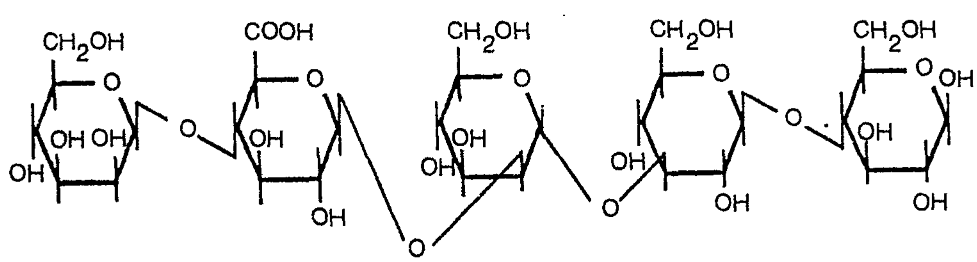

(II)

This structure would require a glucose:mannose:uronic acid molar ratio of 1.0:1.0:0.50.

**Claims**

1. Process for the production of a xanthan gum depolymerising enzyme which comprises culturing *Corynebacterium* strain 20/122 (deposited with NCIB under accession number 11535) under aerobic conditions in a liquid growth medium comprising assimilable sources of nitrogen and essential mineral salts and, as the sole carbon source, xanthan gum, separating the bacterial cells from the reaction mass and recovering the xanthanase from the resultant spent growth medium.

2. Process as claimed in Claim 1 wherein the culture is effected at a temperature between 25° and 40°C and a pH between pH5 and 8.

3. Process as claimed in Claim 2 wherein the culture is effected at a temperature of 30°C and a pH between pH 6.0 and 7.0.

4. Process as claimed in Claim 1, 2 or 3 wherein the recovery of the xanthanase is effected by precipitation through the addition of ammonium sulphate or ethanol.

5. Process as claimed in Claim 4 wherein the xanthanase precipitate is purified by solution in distilled water, dialysis and freeze-drying.

6. Process as claimed in Claim 1, 2 or 3 wherein the xanthanase is recovered by freeze-drying the cell-free spent growth medium.

7. Xanthanase enzyme produced by a process as claimed in any one of the preceding claims, characterised in that it produces four major products on incubation with deacetylated xanthan gum at

30°C and pH 5.6, which can be resolved by chromatography on cellulose thin layer plates using, as solvent, ethyl acetate-pyridine-glacial acetic acid — water (5:5:1:3 by volume), and detected with standard silver nitrate-sodium hydroxide spray reagent, and which are (1) mannose, (2) mannose ketal-linked to pyruvate, (3) an oligosaccharide containing glucose, mannose and uronic acids in the approximate proportions 1.0:0.5:0.34 respectively, and (4) an oligosaccharide containing glucose, mannose and uronic acids in the approximate proportions 1.0:0.84:0.55.

8. Process for the depolymerisation of xanthan gum and/or carboxymethyl cellulose (CMC) in which an aqueous solution of the gum or CMC is reacted with the xanthanase as claimed in Claim 7 at a temperature between 25° and 70°C and a pH between pH 5 and 8.

9. Process as claimed in Claim 8 wherein the reaction is carried out at a temperature of 30°C and a pH between pH 5.5 and 7.

**Revendications**

1. Procédé pour la production d'une enzyme dépolymérisant la gomme xanthane, selon lequel on cultive la souche *Corynebacterium* 20/122 (déposée avec NCIB sous le numéro d'enregistrement 11535) dans des conditions aérobies dans un mileu de culture liquide comprenant des sources assimilables d'azote et des sels minéraux essentiels et, comme seule source de carbone, de la gomme xanthane, on sépare les cellules bactériennes de la masse de réaction et on recueille la xanthanase à partir du milieu de culture usé résultant.

2. Procédé selon la revendication 1, dans lequel la culture est effectuée entre 24°C et 40°C et à un pH compris entre 5 et 8.

3. Procédé selon la revendication 2, dans lequel la culture est effectuée à une température de 30°C et à un pH compris entre 6,0 et 7,0.

4. Procédé selon l'une des revendications 1 à 3, dans lequel le recueil de la xanthanase est effectué par précipitation par addition de sulfate d'ammonium ou d'éthanol.

5. Procédé selon la revendication 4, dans lequel le précipité de xanthanase est purifié par dissolution dans l'eau distillée, dialyse et séchage par congélation.

6. Procédé selon l'une des revendications 1 à 3, dans lequel la xanthanase est recueillie par séchage par congélation du milieu de culture exempt de cellules.

7. Enzyme xanthanase produite par un procédé selon l'une quelconque des revendications précédentes, caractérisée en ce qu'elle produit quatre produits principaux par incubation avec la gomme xanthane désacétylée à 30°C et au pH 5,6, qui peuvent être séparés par chromatographie sur des plaques à couche mince de cellulose en utilisant, comme solvant, un mélange acétate d'éthyle-pyridine-acide acétique glacial-eau (5:5:1:3 en volume) et détectés au moyen d'un réactif pulvérisé normal nitrate d'argent-hydroxyde de sodium, et qui sont (1) du mannose, (2) du mannose lié par cètal à du pyruvate, (3) un oligosaccharide contenant du glucose, du mannose et des acides uroniques dans les proportions approximatives de 1,0:0,5:0,34, respectivement, et (4) un oligosaccharide contenant du glucose, du mannose et des acides uroniques dans les proportions approximatives de 1,0:0,84:0,55, respectivement.

8. Procédé pour la dépolymérisation de gomme xanthane et/ou de carboxyméthylcellulose (CMC) dans lequel une solution aqueuse de la gomme ou de CMC est mise à réagir avec la xanthanase telle que revendiquée dans la revendication 7 à une température comprise entre 25°C et 70°C et à un pH compris entre 5 et 8.

9. Procédé selon la revendication 8, dans lequel la réaction est conduite à une température de 30°C et à un pH compris entre 5,5 et 7.

**Patentansprüche**

1. Verfahren zur Herstellung eines Xanthangummi depolymerisierenden Enzyms, umfassend die Züchtung von Corynebacterium, Stamm 20/122 (hinterlegt bei NCIB unter der Nummer 11535), unter aeroben Bedingungen in einem flüssigen Wachstumsmedium, umfassend assimilierbare Quellen für Stickstoff und essentielle Mineralsalze und als einzige Kohlenstoffquelle Xanthangummi, Abtrennen der Bakterienzellen von der Reaktionsmasse und Gewinnung der Xanthanase aus dem erhaltenen verbrauchten Wachstumsmedium.

2. Verfahren nach Anspruch 1, wobei die Züchtung bei einer Temperatur zwischen 25° und 40°C, und einem pH-Wert zwischen 5 und 8 durchgeführt wird.

3. Verfahren nach Anspruch 2, wobei die Züchtung bei einer Temperatur von 30°C und einem pH-Wert zwischen 6,0 und 7,0 durchgeführt wird.

4. Verfahren nach Anspruch 1, 2 oder 3, wobei die Gewinnung der Xanthanase durchgeführt wird durch Ausfällen durch Zugabe von Ammoniumsulfat oder Ethanol.

5. Verfahren nach Anspruch 4, wobei der Xanthanaseniederschlag gereinigt wird durch Lösen in destilliertem Wasser, Dialyse und Gefriertrocknen.

6. Verfahren nach Anspruch 1, 2 oder 3, wobei die Xanthanase gewonnen wird durch Gefriertrocknen des zellfreien verbrauchten Wachstumsmediums.

7. Xanthanaseenzym, das hergestellt worden ist nach einem Verfahren wie in einem der vorangehenden Ansprüche beansprucht, dadurch gekennzeichnet, daß es bei Inkubation mit deacetyliertem Xanthangummi bei 30°C und pH 5,6 4 Hauptprodukte bildet, die durch Chromatografie auf Cellulose-Dünnschichtplatten aufgetrennt werden können, unter Verwendung von Ethylacetat-Pyridin-Eisessig-Wasser (5:5:1:3 Vol.) als Lösungsmittel und nachgewiesen werden können mit einem Standard-Silbernitrat-Natriumhydroxid-Sprühreagens und die 1) Mannose, 2) Mannoseketal gebunden an Pyruvat, 3) ein Oligosaccharid enthaltend Glucose, Mannose und Uronsäuren in ungefähren Anteilen von 1,0:0,5:0,34 und 4), ein Oligosaccharid enthaltend Glucose, Mannose und Uronsäuren und ungefähren Anteilen von 1,0:0,84:0,55 sind.

8. Verfahren zur Depolymerisierung von Xanthangummi und/oder Carboxymethylcellulose (CMC), bei dem eine wässrige Lösung des Gummis oder von CMC umgesetzt wird mit Xanthanase wie in Anspruch 7 angegeben, bei einer Temperatur zwischen 25 und 70°C und einem pH-Wert zwischen 5 und 8.

9. Verfahren nach Anspruch 8, wobei die Reaktion bei einer Temperatur von 30°C und einem pH-Wert zwischen 5,5 und 7 durchgeführt wird.